# EUROPEAN PATENT SPECIFICATION

(11) **EP 2 810 665 B1**
(45) Date of publication and mention of the grant of the patent: **14.10.2020**
(21) Application number: 13743971.7
(22) Date of filing: 16.01.2013
(51) Int. Cl.: A61L 27/32, A61L 27/54, A61P 19/08

(54) **BIOIMPLANT**
BIOIMPLANTAT
BIO-IMPLANT

(30) Priority: 03.02.2012 JP 2012022205
(43) Date of publication of application: 10.12.2014
(73) Proprietor: Saga University, Saga-shi, Saga 840-8502 (JP); KYOCERA Corporation, Kyoto 612-8501 (JP)
(72) Inventor: MAWATARI, Masaaki, Saga-shi Saga 840-8502 (JP); TSUKAMOTO, Masatsugu, Saga-shi Saga 840-8502 (JP); NODA, Iwao, Kyoto-shi Kyoto 612-8501 (JP)
(74) Representative: Viering, Jentschura & Partner mbB Patent- und Rechtsanwälte
(86) International application number: PCT/JP2013/050661
(87) International publication number: WO 2013/114947

(56) References cited:
- EP-A1- 2 606 916
- WO-A1-2008/029612
- WO-A1-2012/023510
- JP-A- 2011 512 959
- US-A1- 2009 280 156
- US-A1- 2010 286 790
- US-A1- 2011 008 407
- SHIMAZAKI ET AL.: 'In Vivo Antibacterial and Silver-Releasing Properties of NovelThermal Sprayed Silver-Containing Hydr' J BIOMED MATER RES PART B: APPL BIOMATER vol. 92B, 2009, pages 386 - 389, XP055083007
- NODA ET AL.: 'Development of Novel Thermal Sprayed Antibacterial Coatingand Evaluation of Release Properties of Si' J BIOMED MATER RES PART B: APPL BIOMATER vol. 89B, 2008, pages 456 - 465, XP055083008
- ANDO ET AL.: 'Development of antibacterial biomaterials' ORTHOPAEDIC SURGERY AND TRAUMATOLOGY vol. 53, no. 5, 30 April 2010, pages 467 - 475, XP008174650
- MIYAMOTO ET AL.: 'Shinki no Kokinsei Seitai Zairyo no Kaihatsu' ANTIBIOTICS & CHEMOTHERAPY vol. 26, no. 9, 2010, pages 125 - 129, XP008174396
- NODA ET AL.: 'Silver-containing hydroxyapatite coating' JOURNAL OF JAPANESE SOCIETY FOR BIOMATERIALS vol. 29, no. 4, 2011, pages 266 - 270, XP008174305
- NODA ET AL.: 'Gin Gan'yu Hydroxyapatite Yosha Gijutsu no Kaihatsu Dai 4 Ho' DAI 40 KAI NIPPON JINKO KANSETSU GAKKAI PROGRAM-SHOROKUSHU 2010, page 350, XP008174647
- NODA ET AL.: 'Gin Gan'yu Hydroxyapatite Yosha Gijutsu no Kaihatsu Dai 5 Ho' DAI 41 KAI NIPPON JINKO KANSETSU GAKKAI PROGRAM-SHOROKUSHU 2011, page 371, XP008174648
- ANDO ET AL.: 'Ginkei Kokin Seitai Zairyo no Kaihatsu' DAI 34 KAI JAPANESE SOCIETY FOR STUDY OF BONE AND JOINT INFECTIONS PROGRAM. SHOROKUSHU 2011, page 64, XP008174646

## Description

### BACKGROUND OF THE INVENTION

The present invention relates to an antimicrobial bioimplant.

### SUMMARY OF THE INVENTION

Use of bioimplants for treatment of bone injuries/diseases is steadily increasing along with expansion of active and elderly populations. For use as a bone substitute for broken or removed bones or for use as a support to assist a weakened bone, the synthetic bone substitute should form a strong joint or bone together with natural bones and assure the structural integrity thereof. A bone can grow into a neighboring tissue, especially when it is a porous tissue similar to the bone. However, in addition to the growth into porous tissue, the natural bone thus grown into the porous tissue should bind to the bioimplant, forming strong adhesion between them.

An important requirement for fixation of a bioimplant to bone is that the bone grows on and/or into the surface of the bioimplant. Various studies disclose that a calcium phosphate coating on an implant made of cobalt-chromium (Co-Cr) or a titanium (Ti) alloy, for example, a biologic apatite accelerates bone adhesion more quickly than if the implant made of the alloy has a non-coated surface. The biologic apatite Ca₁₀(PO₄)₆(OH)₂ is one of the main compounds which constitute human bone and teeth. The synthesized hydroxyapatite (HA) closely resembles a natural apatite and thus has been used in a study in which HA is used in dental and orthopedic implants. An implant has been produced which is easily integrated with neighboring bones and tissues by coating with HA or other crystalline calcium phosphates after transplantation.

However, although use of the synthetic joints in orthopedics for treatment of degenerative joint diseases is a therapeutic treatment effective for reconstruction of joint function, microbes may proliferate on the surface of the synthetic joints, causing post-operative infection. It is because microbes can adhere to the surface of the synthetic joint and the adhered microbes can form a habitat called biofilm. In such a case, antimicrobial agents (antibiotics) are not effective any more, making it difficult to treat the infection. Moreover if myelitis occurs, it is necessary to remove the synthetic joint and repeat the surgery and there may be possibly a case where the infected limb should be ablated.

Therefore, there are proposed a method of coating a hydroxyapatite film having high crystallinity and large specific surface area, which is suited for impregnation with an antibiotic, by precipitating hydroxyapatite on the surface of an implant and drying the hydroxyapatite, and a therapeutic agent-impregnated implant in which the coating film is impregnated with the antibiotic (Patent Document 1). The bioimplant prepared by the method is suited for impregnation of antibiotics. However, since the coating film has uniform pore size and porosity, it is difficult to perform sustained release of a medicine at a desired rate and thus the medicine tends to be eluted at a fixed rate at a time.

Alternatively, the applicant proposed a method of controlling the rate of releasing an antibacterial or antimicrobial agent by adjusting the evanescence speed of HA by adjusting the crystallinity of the coating layer of calcium phosphate-based material (Patent Document 2)

WO2012/023510 A1 discloses a biological implant, wherein a spray coating comprising a calcium phosphate material is formed on at least a portion of a metal, ceramic or plastic substrate and the silver concentration in the spray coating is 0.02 weight% to 3.00 weight%. JP 2011 512959 A discloses a coating for a medical implant, WO 2008/029612 A1 discloses a bioimplant, and US 2010/286790 A1 discloses an implant made of biocompatible materials.

### [Patent Document]

[Patent Document 1] JP-A No. 2005-506879
[Patent Document 1] JP-A No. 2008-73098

### DETAILED DESCRIPTION OF THE PREFERRED EMBODIMENTS

### [Technical Problems to be Solved]

As described in the above, one of the reasons of post-operative infection is that the microbes form the biofilm on the surface of the implant and this causes the antimicrobial agents to be not effective. Especially, for 24 hours after operation, a risk of the microbial infection is significantly high, because the immune function of a patient is significantly weakened. After that period, the patient slowly recovers own immune function. However, a highly possible state of microbial infection continues over a long period ranging from one week to several weeks after an operation. Especially, a patient having autoimmune disease such as diabetes and further having a weak resistance to the microbial infection (compromised host) has a high risk of microbial infection. Further, a patient who develops infection after implant operation has high infection rates ranging from several times to several tens times in comparison with normal case, when having an operation of replacing implant. Thus, a bioimplant which is capable to inhibit the biofilm formation over a long period of time after an operation is needed. However, it was difficult for the conventional bioimplant to be capable to inhibit the biofilm formation over such a long period of time.

Thus, an object of the present invention is to provide a bioimplant which is capable to inhibit the biofilm formation over a long period of time after an operation.

### [Means to Solve the Problems]

The present invention provides a bioimplant as defined in claim 1. A further embodiment of such a bioimplant is described in claim 2. The present invention further provides a method of producing a bioimplant according to claim 3. The bioimplant according to the present invention was made to overcome the problems above.

### [Effect of the Invention]

Since the bioimplant of the present invention can inhibit the biofilm formation over a long period of time after an operation, a curative effect of antimicrobial agents on infection can be maintained, and thereby decreasing risk of post-operative infection. Further, as used herein, "long period of time" refers to a period in whcih the risk of the post-operative infection is high, and the period ranges from one week to several weeks after an operation.

### [Best Mode for Carrying Out the Invention]

Hereinafter, favorable embodiments of the present invention will be described in detail.

The bioimplant according to the present invention is a bioimplant, comprising a base material of metal, ceramic, or plastic and a thermal spraying film made of a calcium phosphate-based material formed at least partially thereon, the silver concentration in the thermal spraying film being 0.05 wt % to 3.00 wt %.

The bioimplant according to the present invention include metal, ceramic, and plastic implants, such as synthetic bones and fixation devices used for treatment of diseases and injuries, synthetic joints used for reconstruction of lost joint function, and synthetic tooth roots used for reconstruction of teeth.

A metal, ceramic, or plastic material may be used as the base material of the bioimplant. A stainless steel alloy, a cobalt-chromium alloy, titanium, a titanium alloy, alumina, zirconia or the like may be used as the metal, but titanium and titanium alloys are preferable. The titanium alloys for use include alloys of titanium with at least one metal selected from aluminum, tin, zirconium, molybdenum, nickel, palladium, tantalum, niobium, vanadium, platinum and the like. Preferably, it is Ti-6AI-4V alloy. Alternatively, the ceramics for use include, for example, alumina, zirconia, composite alumina-zirconia ceramics and the like. Yet alternatively, the plastics for use include, for example, polyethylenes, fluorine resins, epoxy resins, PEEK resins, Bakelite and the like.

The calcium phosphate-based material for use may be a compound or a mixture of two or more compounds selected from the group consisting of hydroxyapatite, α-tricalcium phosphate, β-tricalcium phosphate, and tetracalcium phosphate. It is preferably hydroxyapatite.

### (Production Method)

The thermal spraying methods used for forming a thermal spraying film of a calcium phosphate-based material include flame spraying method, high-speed flame spraying method, plasma spraying method, and cold spraying method. For example in the case of the flame spraying method, a film is formed on the surface of a base material by melting a thermal spraying material or bringing it close to the melting state by placing it in a gas flame generated with oxygen and a flammable gas and spraying the resulting thermal spraying material on the base material. In the case of the normal flame spraying method, the thermal spraying temperature is about 2700°C and the thermal spraying speed is Mach 0.6. Under normal thermal spraying condition, for example, a thermal spraying powder can be fed with 689 kPa (100 psi) dry air into a gas frame torch generated with 345 kPa (50 psi) oxygen gas and 296 kPa (43 psi) acetylene gas and the resulting powder can be thermally sprayed at a thermal spraying distance of 60 to 100 mm.

The thickness of the thermal spraying film is 5 to 100 µm, preferably 20 to 40 µm, since it is not possible to cover the thermal spraying area entirely when the thickness is less than 5 µm and the adhesion strength of the film declines because of the residual stress during thermal spraying when the thickness is more than 100 µm.

A heat-treatment of the prepared thermal spraying film is carried out. The heat-treatment can increase the crystallinity of a calcium phosphate-based material, and thereby improving the stability of the film. The heat-treatment may be carried out at a temperature range of 400 to 1000 °C for 0.5 to 7 hours under the reduced pressure of 10⁻²Pa or less. It is preferable to carry out at a temperature range of 550 to 850 °C for 1 to 5 hours.

Further, a hydration-treatment of the prepared thermal spraying film after carrying out the heat-treatment is carried out. The hydration-treatment can convert oxyapatite to hydroxyapatite, and thereby stabilizing elution property of silver ion. The hydration-treatment is a step of adding water molecule to material, and, for example, it may be carried out by immersing the thermal spraying film in water at a temperature of 60-100 °C for 10-60 minutes.

It is possible to control the silver concentration in the thermal spraying film by adjusting the amount of the raw silver material blended to the thermal spraying material, i.e., the calcium phosphate-based material. The silver concentration in the thermal spraying film is 0.05 wt % to 3.00 wt %, preferably 0.05 wt % to 2.50 wt %, more preferably 0.05 wt % to 1.00 wt %, and more preferably 0.1 wt % to 1.00 wt %. It is because the antimicrobial action is not sufficient when the silver concentration is less than 0.05 wt %. Alternatively when it is more than 3.00 wt %, the implant may become toxic to tissues and organs in the body. According to literature, use of a great amount of silver leads to Argylia disease (disease leading to graying in color of the entire skin), decrease of leucocytes, and damage to liver and kidney. Our studies also showed that there are deformation of cells and inhibition of neonatal bone formation when the silver concentration is more than 3.00 wt %.

An example of the bioimplant according to the present invention is a synthetic joint consisting of a stem which is a bone contact portion inserted into the bone and a neck unit formed on the top end of the stem for fixation of bone head ball, wherein at least part of the bone contact portion is covered with a thermal spraying film of a calcium phosphate-based material and the silver concentration in the thermal-spray film is 0.05 wt % to 3.00 wt %. The synthetic joint is preferably made of titanium or a titanium alloy.

### [Examples]

### Experiment 1 - Reference example

### (Sample Preparation)

Hydroxyapatite containing a particular amount of silver oxide was sprayed onto one side of a pure titanium plate with a size of 50 mm × 50 mm × 2 mm by flame spraying method, to form a thermal-spray film having a thickness of about 40 µm.

The flame spraying was carried out by introducing, with 689 kPa (100 psi) dry air, the thermal spraying powder into a gas frame torch generated with 345 kPa (50 psi) oxygen gas and 296 kPa (43 psi) acetylene gas and spraying the fused powder at a thermal spraying distance of 60 to 100 mm.

### (Measurement of Silver Concentration)

After sufficient drying at 100°C, each sample was weighed and then dissolved in a nitric acid solution (5 mL of nitric acid and 50 mL of purified water) while heating. The silver concentration in the film was determined by measuring the silver concentration in the solution quantitatively by ICP emission spectrophotometric analysis. Then, the sample after removal of the film by solubilization was dried sufficiently and weighed again, and the film weight was calculated from the difference in weight from the sample before solubilization. The silver concentration in film (wt%) was calculated by dividing the amount of silver in film by the weight of the film. The silver concentration of the film in this experiment was 0.3 wt%.

### (Test for Antimicrobial Activity)

As a test for antimicrobial activity, an evaluation of performance of inhibiting biofilm formation was carried out. Methicillin-resistant Staphylococcus aureus was adhered to samples, and then the samples was immersed in a fetal bovine serum retained at a temperature of 37°C and was cultivated under flow condition by stirring at 60 rpm. After culture for one week and two weeks, biofilm formed on the film was stained by fluorescent staining and biofilm coverage on the film was determined and morphology thereof observed by fluorescence microscope. Further, the biofilm coverage was determined from a surface area ratio of a fluorescence emission area obtained by using image analysis software.

### Experiment 2 - Reference example

A sample was prepared in a similar manner with experiment 1 except that Hydroxyapatite containing no silver oxide was used, and supplied to the test for antimicrobial activity.

### (Result)

Table 1 shows the biofilm coverage. The biofilm coverage of the samples of one-week culture and two-week culture in the experiment 1 became lower than those of the control samples, indicating that the samples of the experiment 1 shows inhibiting the biofilm formation.

**Table1**

| | Biofilm coverage (%) | |
|---|---|---|
| | After culture for 1 week | After culture for 2 weeks |
| Experiment 1 | 1.3 | 29.9 |
| Experiment 2 | 5.7 | 48. 9 |

### Experiment 3 - Reference example

This experiment was carried out to reconfirm the effect of the silver concentration in the film, and the samples having silver concentrations of 0, 0.05, 0.1, 0.3 wt% were prepared.

### (Sample Preparation)

Hydroxyapatite containing a particular amount of silver oxide was sprayed onto one side of a pure titanium plate with a diameter of 14 mm and a thickness of 1 mm by flame spraying method, to form a thermal-spray film having a thickness of about 40 µm. The samples having silver concentrations of 0, 0.05, 0.1, 0.3 wt% were prepared by adjusting the amount of the silver oxide blended to the thermal spraying material,

The flame spraying was carried out in a similar manner as described in Experiment 1.

### (Measurement of Silver Concentration)

The measurement of silver concentration was carried out in a similar manner as described in Experiment 1.

### (Test for Antimicrobial Activity)

As a test for antimicrobial activity, a test of inhibiting biofilm formation was carried out. The test was carried out in a similar manner as described in Experiment 1 except that a culture medium was replaced every fourth day to avoid the culture medium of fetal bovine serum from being saturated with a bacteria.

### (Result)

Table 2 shows the results of measurements of the biofilm coverage. The biofilm coverage of the samples of one-week culture and two-week culture in this experiment became lower in proportion to the silver concentration in the film. This indicated that biofilm formation is inhibited as the silver concentration in the film increases. Further, considering the results of this experiment and empirical knowledge on the bacterial growth, it is estimated that the samples maintain an effect of inhibiting biofilm formation for a long period time, such as about four weeks when the silver concentration is 0.05%, about six weeks when the silver concentration is 0.1%, and about ten weeks when the silver concentration is 0.3%.

**Table 2**

| | Biofilm coverage % | |
|---|---|---|
| Silver concentration in film (wt%) | After one-week culture | After two-week culture |
| 0 | 28.6 | 55.0 |
| 0.05 | 7.5 | 37.1 |
| 0.1 | 5.3 | 21.4 |
| 0.3 | 3.6 | 9.3 |

Since the bioimplant of the present invention is capable to inhibit the biofilm formation over a long period of time after an operation, the risk of post-operative infection can be significantly decreased. Especially, a large effect can be expected when applying to patients having high risk of infection, for example, to a compromised host or a patient who develops infection after implant operation and have an operation of replacing implant.

## Claims

1. A bioimplant, comprising a base material of metal, ceramic, or plastic and a thermal spraying film of a calcium phosphate-based material formed at least partially thereon, and the bioimplant being subjected to a heat-treatment at a temperature range of 400 to 1000 °C under the reduced pressure of 10⁻² Pa or less and then to a hydration-treatment, the silver concentration in the thermal-spray film being 0.05 wt % to 3.00 wt %, wherein the thermal spraying film is hydrated and hydroxyapatite is present on the surface of the thermal spraying film.

2. The bioimplant according to claim 1, wherein said calcium phosphate-based material is a compound or a mixture of two or more compounds selected from the group consisting of hydroxyapatite, α-tricalcium phosphate, β-tricalcium phosphate, and tetracalcium phosphate.

3. A method of producing a bioimplant comprising a base material of metal, ceramic, or plastic and a thermal spraying film of a calcium phosphate-based material formed at least partially thereon and having a silver concentration of 0.05 wt % to 3.00 wt %, wherein hydroxyapatite is present on the surface of said thermal spraying film, the method comprising:
spraying the calcium phosphate-based material containing raw silver material onto the base to form the thermal spraying film; and
subjecting the bioimplant to a heat-treatment at a temperature range of 400 to 1000 °C under the reduced pressure of 10⁻² Pa or less and then to a hydration-treatment.

## Patentansprüche

1. Bioimplantat, aufweisend ein Grundmaterial aus Metall, Keramik oder Kunststoff und einen thermisch gesprühten Film aus einem zumindest teilweise darauf gebildeten Calciumphosphat-basierten Material, und wobei das Bioimplantat einer Wärmebehandlung in einem Temperaturbereich von 400 bis 1000 °C unter dem reduzierten Druck von 10⁻² Pa oder weniger und dann einer Hydratationsbehandlung unterzogen wird, wobei die Silberkonzentration in dem thermisch gesprühten Film 0,05 Gew.-% bis 3,00 Gew.-% beträgt, wobei der thermisch gesprühte Film hydratisiert ist, und wobei Hydroxylapatit auf der Oberfläche des thermisch gesprühten Films vorhanden ist.

2. Bioimplantat nach Anspruch 1, wobei das Calciumphosphat-basierte Material eine Verbindung oder eine Mischung von zwei oder mehr Verbindungen ist, ausgewählt aus der Gruppe bestehend aus Hydroxylapatit, α-Tricalciumphosphat, β-Tricalciumphosphat und Tetracalciumphosphat.

3. Verfahren zum Herstellen eines Bioimplantats aufweisend ein Grundmaterial aus Metall, Keramik oder Kunststoff und einen thermisch gesprühten Film aus einem zumindest teilweise darauf gebildeten Calciumphosphat-basierten Material und mit einer Silberkonzentration von 0,05 Gew.-% bis 3,00 Gew.-%, wobei Hydroxylapatit auf der Oberfläche des thermisch gesprühten Films vorhanden ist, das Verfahren aufweisend:
Sprühen des Calciumphosphat-basierten Materials enthaltend rohes Silbermaterial auf das Grundmaterial, um den thermisch gesprühten Film zu bilden; und
Unterziehen des Bioimplantats einer Wärmebehandlung in einem Temperaturbereich von 400 bis 1000 °C unter dem reduzierten Druck von 10⁻² Pa oder weniger und dann einer Hydratationsbehandlung.

## Revendications

1. Bioimplant, comprenant un matériau de base en métal, céramique ou plastique et un film de projection thermique d'un matériau à base de phosphate de calcium formé au moins partiellement sur celui-ci, et le bioimplant étant soumis à un traitement thermique dans une plage de température de 400 à 1000 °C sous une pression réduite de 10⁻² Pa ou moins et ensuite à un traitement d'hydratation, la concentration en argent dans le film de projection thermique étant de 0,05°% en poids à 3,00°% en poids, dans lequel le fil de projection thermique est hydraté et de l'hydroxyapatite est présente à la surface du film de projection thermique.

2. Bioimplant selon la revendication 1, dans lequel ledit matériau à base de phosphate de calcium est un composé ou un mélange de deux ou plusieurs composés choisis dans le groupe constitué par l'hydroxyapatite, le phosphate α-tricalcique, le phosphate β-tricalcique et le phosphate tétracalcique.

3. Méthode de production d'un bioimplant comprenant un matériau de base en métal, céramique ou plastique et un film de projection thermique d'un matériau à base de phosphate de calcium formé au moins partiellement sur celui-ci et ayant une concentration en argent étant de 0,05°% en poids à 3,00°% en poids, dans lequel de l'hydroxyapatite est présente à la surface dudit film de projection thermique, la méthode comprenant :
projeter le matériau à base de phosphate de calcium contenant le matériau d'argent brut sur le matériau de base pour former le film de projection thermique ; et
soumettre le bioimplant à un traitement thermique dans une plage de température de 400 à 1000 °C sous la pression réduite de 10⁻² Pa ou moins et ensuite à un traitement d'hydratation.
